# EUROPEAN PATENT APPLICATION

(11) **EP 1 329 483 A2**
(43) Date of publication of application: **23.07.2003**
(21) Application number: 02028436.0
(22) Date of filing: 19.12.2002
(51) Int. Cl.: C09C 1/00

(54) **Cosmetic body pigment and process for preparing the same**

(30) Priority: 22.01.2002 JP 2002013009
(71) Applicant: MERCK PATENT GmbH, 64293 Darmstadt (DE)
(72) Inventor: Yukitaka, Watanabe, Iwaki-shi, Fukushima-ken 970-8035 (JP)

(57) **Abstract**

A cosmetic body pigment having a good skin feel, no blue color, a high soft focusing effect (low viewing angle dependency of reflected light intensity), a natural color tone against the skin, and a moderate hiding power (wrinkle hiding effect) is provided. The cosmetic body pigment is characterized in that TiO₂ fine particles are deposited separately with form of mono layer on the surface of a thin-platelet like substrate.

## Description

### [Field of the Invention]

The present invention relates to a cosmetic body pigment and a process for preparing the same.

### [Prior Art]

Pearl pigments (e.g., titanium oxide coated mica) are partly used for a body pigment for cosmetic products because of their good skin feel. For pearl pigments, forming an uniform optical layer is required for the purpose of pearlescence generation, and therefore, smaller sizes and higher densities of TiO₂ particles are preferable, which are generally less than 100 nm. It is considered that the adhesion of a TiO₂ particle onto the surface of mica is caused by a physical force (van der Waals forces). However, for the use in cosmetic body pigments, the generation of pearlescence is not preferable because it causes glitter. In this respect, simply decreasing the amount of coating is considered to be a method for reducing the pearl luster; however, this will result in a blueing effect because of the fine particle size of the TiO₂ for the coating as described above and therefore this is not preferable.

In recent years, there is a demand for a cosmetic body pigment that has such features: (1) a good skin feel such as shipping property and adhesion property, (2) reduced blue color which shows unhealthy hue, (3) a low viewing angle dependency of reflective light (soft focusing effect), and (4) an appropriate hiding power (wrinkle hiding effect) based on semi-transparency and natural color tone to the skin, while maintaining a reduced luster without pearlescence.

Various pigments based on a thin-platelet like substrate have been developed and disclosed especially for cosmetic body pigments such as compact cakes and foundation creams due to the fact they possess a good shipping property owing to their shapes and covering force for the skin (for example, JP-A-05-287212, JP-A-03-181411, JP-A-63-254169, etc.). However, these do not satisfy the above described targeted functions at once. For example, JP-A-09-132514 discloses a thin-platelet like powder for use in a cosmetics with a good skin feel wherein fine particles of an inorganic compound are deposited on a thin-platelet like substrate by electro static interaction thereby allowing the fine particles to move on and drop off the surface thereof; its purpose is the shielding of ultraviolet rays and the fine particles are uniformly dispersed and deposited substantially in a single layer. Consequently, this thin-platelet like fine powder is intended specifically for a good skin feel without expecting color effect and the fine particles are not deposited separately.
JP-A-11-199441 discloses a powder cosmetic comprising mica coated with rutile type TiO₂ fine particles; the aim of which is to provide an ultraviolet shielding effect while maintaining pearlescence, and is therefore opposed to the present invention in terms of the effect (low luster) to be achieved.

### [Problem to be solved by the Invention]

Therefore, it is an object of the present invention to provide a cosmetic body pigment which has a good skin feel, no blue color, a high soft focusing effect (a low viewing angle dependency of reflected light intensity), a natural color tone against the skin, and an appropriate hiding power(wrinkle hiding effect).

### [Means for solving the Problems]

In order to solve the above mentioned problems, the inventors of the present invention have carried out eager investigations on a cosmetic body pigment based on a thin-platelet like substrate and TiO₂ fine particles consequently completing the present, invention by discovering that these problems can be solved by making the TiO₂ fine particles deposited keeping appropriate distance with each other on the surface of the thin-platelet like substrate.
Thus, the present invention relates to a cosmetic body pigment, characterized in that TiO₂ fine particles are deposited in a scattered condition to form a single layer on the surface of a thin-platelet like substrate.

The present invention also relates to the above described cosmetic body pigment characterized in that the mean particle diameter of the TiO₂ fine particles is 150 to 450 nm.
The present invention further relates to the above described cosmetic body pigment characterized in that the particle diameters of the TiO₂ fine particles are not smaller than 100 nm.
The present invention still further relates to the above described cosmetic body pigment characterized in that the TiO₂ fine particles are either untreated or surface treated with one or more hydroxides and/or oxide hydrates of which the principal component is an oxide of one or more metals selected from the group consisting of Si, Al, Zn, and Fe.

The present invention still further relates to the above described cosmetic body pigment characterized in that the amount of the TiO₂ fine particles is 15 to 55 wt% of the total amount of the cosmetic body pigment.
The present invention still further relates to the above described cosmetic body pigment characterized in that a mean center-to-center distance between a TiO₂ particle and its six neighboring TiO₂ fine particles is not smaller than one and a half times the mean particle diameter of the TiO₂ fine particles.
The present invention still further relates to the above described cosmetic body pigment characterized in that a mean particle diameter of the thin-platelet like substrate is 0.5 to 50 µm and a mean thickness is 0.01 to 1 µm. The present invention still further relates to the above described cosmetic body pigment characterized in that the thin-platelet like substrate is mica.

The present invention still further relates to the above described cosmetic body pigment characterized in that the TiO₂ fine particles adhere to the surface of the thin-platelet like substrate by a surface electrical charge attractive force.
The present invention still further relates to a process for preparing a cosmetic body pigment comprising following steps;
suspending thin-platelet like substrates in water and adding TiO₂ fine particles thereto while stirring;
depositing the TiO₂ fine particles onto the surface of a thin-platelet like substrate by changing the pH value of the suspension containing the thin-platelet like substrates and the TiO₂ fine particles; and
collecting solids components of said suspension by filtering and washing followed by drying them.

The present invention further relates to a cosmetic composition containing the above mentioned cosmetic body pigments.
The present invention also relates to a resin composition containing the above mentioned cosmetic body pigments.

The cosmetic body pigment of the present invention simultaneously exhibits the following features based on the mechanisms contemplated as described below.

### 1) Skin feel (shipping property, adhesion property)

The TiO₂ fine particles on the surface of a thin-platelet like substrate prevent the direct contact among the planes of the thin-platelet like substrate, and since the fine particles are deposited keeping appropriate distance with each other, the direct contact between the planes of the thin-platelet like substrate and the skin is reduced thereby facilitating the easy contact between the TiO₂ fine particles thereon and the skin. As the result of this, the contact area of the cosmetic body pigment with respect to the skin substantially decreases compared with the case where no particle is deposited, and thereby friction is reduced increasing shipping property. Because the TiO₂ fine particles are scattered on the surface of the thin-platelet like substrate, the TiO₂ fine particles readily come into the depressions of the skin thereby improving the adhesion property to the skin.

### 2) Reduced blue color

Since the size of the TiO₂ fine particles of the present invention is suitable for reflecting visible wavelength range of light, the effect of ultraviolet scattering is small and therefore the blue color will not stand out. Also, since TiO₂ fine particles with moderate sizes are scattered, no optical interprence layer is formed and therefore the pearl luster will hardly generates.

### 3) Soft focusing effect (low viewing angle dependency of light reflection)

Since the TiO₂ fine particles on the surface of the thin-platelet like substrate are deposited keeping appropriate distance one another, incident light from respective angle tends to reflect back into all directions as scattered light from the surface of the fine particle and thus the viewing angle dependency of reflected light is reduced resulting in a good soft focusing effect.

### 4) Natural color tone to the skin

The TiO₂ fine particles deposited keeping appropriate distance one another on the surface of a thin-platelet like substrate lead to a mixed condition of two portions: a scattered reflection light portion from the surfaces of the TiO₂ fine particle, and a no-scattered reflection light portion from the surface of thin-platelet like substrate which is no coated part. Thus a semi-transparent portion due to the material properties of the thin-platelet like substrate and a scattering portion which scatters natural light due to TiO₂ fine particles are moderately mixed and, as the result, natural scattered light matched to the skin is obtained.
Through these mechanisms, the present invention provides a solution to the existing problems achieving the above described effects at once.

Moreover, according to a process for preparing the cosmetic body pigment of the present invention, it is possible to make almost all amount of TiO₂ fine particles added to the surface of the thin-platelet like substrate be deposited keeping appropriate distance one another spacing in a scattered condition, and this preparation can be readily performed.

### [Embodiments of the Invention]

The present invention will be described in more detail in the following.
For the thin-platelet like substrate used in the present invention, any material generally used for the substrate to prepare a cosmetic body pigment can be used. They include, for example, natural mica, synthetic mica, talc, sericite, silica, thin-plate-like barium sulfate, alumina flake, and so forth. Out of these materials, mica is preferable because of its availability and semi-transparency, which allows the generation of color tone natural to the skin. More concretely, muscovite, phlogopite, synthetic mica, fluorine tetravalent silicon mica are preferable.

The mean particle diameter of the thin-platelet like substrate is preferably 0.5 to 50 µm and more preferably 1 to 20µm considering the shipping property, agglomeration of the thin-platelet like substrate itself, glitter and skin feel as a cosmetics.
The mean thickness of the thin-platelet like substrate of the present invention is moderately selected from the balance between the transparency and hiding power (semi transparency), and is preferably 0.01 to 0.1µm when considering the mechanical strength to preserve the thin-platelet like shape of the thin-platelet like substrate, transparency of the thin-platelet like substrate itself, and natural light scattering effect. In another word, a thickness suitable for providing semi-transparency and generating a natural color tone for the skin is selected. Accordingly, the mean thickness is appropriately selected considering the balance between the transparency and the hiding power, and the intrinsic hue of the thin-platelet like substrate.

The TiO₂ fine particle used in the present invention may be either anatase type or rutile type, but its size is different from the ones intended for general ultraviolet shielding, and is preferably as follows.
The mean diameter of the TiO₂ fine particles is preferably 150 nm to 450 nm, and more preferably 180 to 300 nm. When the mean diameter meets the above mentioned requirement, it is preferable because visible light is scattered and an appropriate hiding power is maintained, and it is further desirable to make the distribution as narrow as possible. For example, TiO₂ fine particles with an mean diameter of 250 nm effectively exhibit light scattering in the visible light range.

The particle diameter of the TiO₂ fine particle is preferably larger than and equal to 100nm. When it is larger than 100nm, the generation of blue coloring is reduced and thereby scattering effect of visible light can be obtained to generate a natural color tone on the skin.
Also, to exert the soft focus effect more effectively, the shape of the TiO₂ fine particle is best to be spherical.

Therefore, when the TiO₂ fine particles are deposited separately in a single layer on the surface of the thin-platelet like substrate, visible light is scattered thereby reducing blue color and an appropriate hiding power is maintained, and therefore, soft focus effect is effectively displayed and thereby an body pigment suitable for cosmetics is achieved.
On the contrary, when the size of the TiO₂ fine particle is small, the scattering of visible light is gradually reduced thereby facilitating transmission, and scattering in the ultraviolet range occurs causing the blue color tone to appear. Also, optical reflections (luster and interference) start to occur. Further, in this case, TiO₂ fine particles tend to aggregate together thereby making it difficult to maintain proper distance among TiO₂ fine particles.

Therefore, when smaller TiO₂ fine particles, whose mean diameter is out of the range of the present invention, are deposited on the surface of thin-platelet like substrate, the whole body pigment itself exhibits blue color and also the TiO₂ fine particles aggregate easily together forming unevenness or lumps, and this will make it difficult to make the TiO₂ fine particles deposited separately the TiO₂ fine particles keeping a some constant distance among the particles, and therefore is not preferable.
Thus, this will not satisfy the target of the present invention, which is to provide a pigment having no blue color and a high skin feel.
When the mean particle diameter is large, the TiO₂ fine particles are resistant to the aggregation among themselves, but their adhesion power to the surface of the thin-platelet like substrate is reduced tremendously and makes the TiO₂ fine particles tend to peel off easily from the thin-platelet like substrate. Therefore, this will make it difficult to prepare cosmetic body pigments according to the present invention in which TiO₂ fine particles are deposited separately.

The TiO₂ fine particle used in the present invention is prepared by means of known methods. For example, it is possible to prepare them from titanyl sulfate and titanium tetrachloride by a thermal hydrolysis method or a neutralizing hydrolysis method. It is also possible to form a rutile type by selecting calcining temperature or adding auxiliary agents. The preparation can also be performed by a sol-gel method using titanium alcoholates.

In the present invention, the word ' TiO₂ fine particle' does not exclusively mean a complete titanium dioxide, but includes hydroxides and hydrates whose principal component is a titanium dioxide and thus generically refers to an unitary mixed condition.
In the present invention, thus obtained TiO₂ fine particles may be used untreated, or may be used with a surface treatment using hydroxides and/or hydrate oxides whose principal component is at least one of the oxides of Si, Al, Zn, or Fe. Here, 'hydroxides and/or hydrate oxides whose principal component is at least one of the oxides of Si, Al, Zn, or Fe' means a state in which oxides, hydroxides and hydrates of those metals are intermixed in an unitary state. This surface treatment is generally applied by the amount less than 5 wt%. In the present invention, TiO₂ fine particles of a commercially available pigment class may also be used and this is preferable in terms of convenience. For example, they are exemplified by 'TIPAQUE CR-50' (mean particle size 250 nm), manufactured by ISHIHARA SANGYO Ltd and 'TITANIX JA-C' (mean particle size 180nm), manufactured by TAYCA CORPORATION.

Also preferably, an mean of the center-to-center distances between TiO₂ fine particles among six neighboring particles is not smaller than one and a half times the mean particle diameter of the TiO₂ fine particles.
The mean center-to-center distance among six neighboring particles is specifically obtained in such a way that nearest neighboring six particles with respect to a focused particle are selected and their distances are measured to calculate a mean on a SEM photography, and then another focused particle is arbitrary selected to measure a mean distance of its nearest six neighbors as before and similar measurements are repeated on 20 focused particles to calculate means based on the measurements.
Distribution of the TiO₂ fine particles deposited separately keeping appropriate distance within the above mentioned range will reduce the chances of contact among TiO₂ fine particles on a thin-platelet substrate and, because of this, when they are used as a cosmetics, the thin-platelet substrate comes into contact with the skin through the TiO₂ fine particles and this will favorably affect the spreadability of the cosmetics thereby improving the skin feel.

The amount of the TiO₂ fine particles of the present invention is preferably 15 to 55 wt% of the total amount of the cosmetic body pigment, and more preferably 20 to 50 wt%.
When a greater amount of the TiO₂ fine particles are used, the probability of contact among the TiO₂ fine particles increases thereby causing them to form a block, and this makes it difficult to maintain predetermined distance between the centers of the TiO₂ fine particles, and thereby skin feel is decreased. Also since the exposed area on the surface of the thin-platelet like substrate base decreases, a moderate balance between the transparency and the hiding power can not be achieved and thus natural color tone to the skin is not achieved either. Furthermore, in production, more TiO₂ fine particles will aggregate and particles which do not adhere to the substrate increase. On the other hand, when a smaller amount of TiO₂ fine particles are used, the probability of direct contact among thin-platelet like substrates increases, and this leads to a decline of the shipping property to the skin and also to a decline of the skin feel. Thus, intrinsic properties and hues of the thin-platelet like substrate stand out as they are, making it difficult to achieve the object of the present invention, which is to generate a natural color tone to skin. Therefore, the amount of the TiO₂ fine particles to be used in the present invention is determined within the above described range by considering the relationship between the density and the surface area of the thin-platelet like substrate.

For the process for preparing the cosmetic body pigment of the present invention, either a dry processing or a wet processing may be adopted. For the dry process for preparing, a mechanochemical method (for example, JP-A-5-214257) may be adopted. Particularly, in the present invention, a wet processing is preferably adopted to make TiO₂ fine particles deposited keeping appropriate distance each other and to make the spacing as uniform as possible.

In particular, for process of preparation adopted in the present invention, the following method is recommended.
First, thin-platelet like substrate is suspended in water, and to this suspension, TiO₂ fine particles with a predetermined particle diameter are added without any pretreatment or after dispersing the TiO₂ fine particles in water making a suspension. Since the smaller the size of TiO₂ fine particles, the more likely the fine particles aggregate, it is desirable to prepare a TiO₂ fine particle suspension in advance and use it. Then, TiO₂ fine particles are deposited on the surface of the thin-platelet like substrate by changing the pH value of this suspension, and when they are surface treated with aluminum etc., an acid water solution is used by adjusting pH below 3 while stirring. Subsequently, pH is adjusted to a neutral point and solids components are filtered, washed and then dried. According to the above mentioned method, it is possible to make almost all the added TiO₂ fine particles deposited separately on the surface of the thin-platelet like substrate and making them deposited thereon satisfactorily, and therefore it is preferable.

The mechanism with which TiO₂ fine particles are deposited separately on the surface of thin-platelet like substrate is not necessarily clear; it is considered that a surface electrical charge attractive force acts between the surfaces of a TiO₂ fine particle and thin-platelet like substrate through the control of pH at the hydroxyl group and/or hydrate groups which exist on the surface of the Tio₂ fine particles. Moreover, in the case where a surface treatment is applied, especially in the case of Al, Zn, and Fe, part of their hydroxyl groups and/or hydrate groups dissolved and a surface electrical attractive force acts between the exposed area of the TiO₂ fine particle and the thin-platelet like substrate as described above, and further part of the surface treated metal salts which have dissolved precipitates while resetting the pH value to neutral and acts as an adhesive.

The cosmetic body pigment of the present invention can be used for cosmetic compositions, resin compositions, and so forth, which are described in more detail below.

### (1) cosmetic composition

The cosmetic body pigment of the present invention may be used after applying various surface treatments (e.g., silan coupling treatment, titanium coupling treatment, fluoro compound treatment, hydrogenpolysiloxane treatment, perfluoroesterphosphate treatment, etc.) in advance.
Examples of use in cosmetics include make-up, hair care products, etc. For example, the pigments can be used in gel, lipstick, foundation (including emulsion, liquid, oil-type, etc.), rouge, mascara, nail enamel, eyebrow pencil, eye shadow, eye liner, hair products, etc.
The content of the cosmetic body pigment of the present invention for the cosmetic composition can be arbitrarily selected as appropriate. For example, for foundations 1-50 wt%, for eye shadow 1-80 wt%, for lipstick 1-40 wt%, for nail enamel 0.1-20 wt% can be mentioned.

In the present invention, the mixtures other than the cosmetic body pigment of the present invention are exemplified as follows.
Examples of inorganic pigments, body pigments, and others include: titanium dioxide, calcium carbonate, clay, talc, barium sulfate, white carbon, chromium oxide, zinc oxide, zinc sulfide, zinc powder, metal powder pigments, iron black, yellow iron oxide, red iron oxide, chrome yellow, carbon black, molybdate orange, Prussian Blue, ultramarine blue, cadmium type pigments, fluorescent pigments, soluble azo dyes, insoluble azo dyes, condensed azo dyes, phthalocyanine pigments, condensed polycyclic pigments, composite oxide pigments, graphite, mica (e.g., muscovite, phlogopite, synthetic mica, fluorine tetra silicon mica, etc.), metal oxide coated mica (e.g., titanium oxide coated mica, titanium dioxide coated mica, (hydrated) iron oxide coated mica, mica coated with iron oxides and titanium oxides, mica coated with lower ordered titanium oxides), metal oxide coated graphite (e.g., titanium dioxide coated graphite, etc.), thin-platelet-like alumina, metal oxide coated thin-platelet like alumina (e.g., titanium dioxide coated thin-platelet like alumina, iron oxide coated thin-platelet-like alumina, Fe₂O₃ coated thin-platelet like alumina, Fe₃O₄ coated thin-platelet like alumina, interference color metal oxide coated thin-platelet like alumina, etc.), MIO, sericite, magnesium carbonate, silica, zeolite, hydroxyapatite, chromium oxide, cobalt titanate, glass beads, nylon beads, silicone beads, etc.

Examples of organic pigments include red nos. 2, 3, 102, 104, 105, 106, 201, 202, 203, 204, 205, 206, 207, 208, 213, 214, 215, 218, 219, 220, 221, 223, 225, 226, 227, 228, 230-1, 230-2, 231, 232, 405; yellow nos. 4, 5, 201, 202-1, 202-2, 203, 204, 205, 401, 402, 403, 404, 405, 406, 407; green nos. 3, 201, 202, 204, 205, 401, 402; blue nos. 1, 2, 201, 202, 203, 204, 205, 403, 404; orange nos. 201, 203, 204, 205, 206, 207, 401, 402, 403; brown no. 201; violet nos. 201, 401; black no. 401.

Examples of natural colorants include salol yellow, carmine, β-carotin, hibiscus color, capsaicin, carminic acid, laccaic acid, gurcumin, riboflavin, shikonin, etc.
Examples of other components include fats and oils, waxes, surfactants, oxidation inhibitors, UV absorbers, vitamins, hormones, squalanes, liquid paraffins, palmitic acids, stearic acids, bees wax, myristyl myristate etc., organic solvent of acetone, toluene, butyl acetate, acetic esters etc., antioxidants, antiseptic agents, polyhydric alcohols, perfumes, etc.
The above described effects are achieved by combining these components and the cosmetic body pigment of the present invention.

### (2) Resin composition of the present invention

The resin compositions may contain the cosmetic body pigment of the present invention.
To improve the dispersibility in the resin, the cosmetic body pigment of the present invention may be used by treating with a silane coupling agent or a titanate coupling agent.
The preferable content of the cosmetic body pigment of the present invention is, for example, 0.1 to 50 wt% of the resin component, and more preferably 0.1 to 20 wt%, and still more preferably 0.1 to 5 wt%.

For the resin components of the resin composition, thermoplastic resins and thermosetting resins may be used.
Examples include, polyethylene, chlorinated polyethylene, polypropylene, methylpentene, AAS, ABS, ACS, AES, AS, EEA, ethylene-vinyl acetate copolymer, EVOH, ionomer, methacrylate, PCT, polystyrene, vinyl chloride, vinylidene chloride, thermoplastic elastomer, thermoplastic polyurethane elastomer, diallyl phthalate, epoxy, melamine, phenol, urea, polyester, polyurethane, silicone, polyamide, polybutylene terphthalate, polyethylene terephthalate, polyoxymethylene, polycarbonate, polyphenylene ether, unsaturated polyester, fluororesin, polyetheretherketone, polyetherketone, crystalline liquid polymer, polyphenylene sulfide, polyarylate, polyaryl sulfone, polyether-imide, polyethersulfone, polysulfone, polyamide-imide, polyamide, cellulose acetate, polybutadiene, polydicyclopentadiene, polyketone, polyphthalamide, EMAA, polybutene, polyacrylonitrile, polyacetal, polyvinyl acetal, amino-, alkyd-, biodegradable plastics(e.g., bacteria cellulose, biopolyester as the microbial based material, and polycaprolactam, polyethylene succinite, polyactic acid as the chemical synthesis based material, and starch, cellulose acetates as the natural matters), and copolymers, block copolymer, graft copolymer of the above, natural rubber, synthetic rubber, silicone rubber, etc.

Examples of mixtures and admixtures include pigments, dyes, paints, crosslinking agents, vulcanizers, vulcanizing accelerators, oxidation inhibitors, age resistors, plasticizers, ultraviolet absorbers, light stabilizers, fillers, reinforcers, slip additives, flame-retardants, antistatic additives, foaming agents, curing agents, modifying agents, etc.

For example, pigments other than the cosmetic body pigment of the present invention include titanium dioxide, calcium carbonate, clay, talc, barium sulfate, white carbon, chromium oxide, zinc oxide, zinc sulfide, zinc powder, metal powder pigments, iron black, yellow iron oxide, red iron oxide, chrome yellow, carbon black, molybdate orange, Prussian Blue, ultramarine, cadmium pigments, fluorescent pigments, soluble azo dyes, insoluble azo dyes, condensed azo dyes, phthalocyanine pigments, condensed polycyclic pigments, composite oxide pigments, graphite, mica (e.g., nuscovite, phlogopite, synthetic mica, fluorine tetravalent silicon mica, etc.), metal oxide coated mica (e.g., titanium oxide coated mica, titanium dioxide coated mica, (hydrated) iron oxide coated mica, mica coated with iron oxides and titanium oxides, mica coated with lower order titanium oxides), metal oxide coated graphite (e.g., titanium dioxide coated graphite, etc.), thin-platelet-like alumina, metal oxide coated thin-platelet like alumina (e.g., titanium dioxide coated thin-platelet like alumina, iron oxide coated thin-platelet like alumina, Fe₂O₃ coated thin-platelet like alumina, Fe₃O₄ coated thin-platelet like alumina, interference color metal oxide coated thin-platelet like alumina, etc.), MIO, etc.

The moldings of this resin composition include: for example, resin moldings, laminated products, films (agriculture uses, food product uses, building dressing uses, etc.), sheets (agriculture uses, food product uses, building dressing uses), packaging media, food wrapping sheets or films, and its application areas include: various retainers, electric and electronics devices, electric household appliances, OA/AV equipment parts, rubber products, automobile parts, cosmetics, dressing boards, corrugated panel materials, building materials, wall papers, floor covering materials, wall paper materials, bands, tires, caps, etc. The resin composition also can be used for laser marking. The molding and process for preparing this resin composition include injection molding, cast molding, extrusion molding, transfer molding, inflation molding, stretch molding, vacuum molding, blow molding, calendaring process, lining process, laminate molding, slash molding, transfer molding, paste molding, etc.

Hereinafter, the present invention will be shown in more detail with reference to embodiments, which are intended to show examples and will not limit the present invention.

### EXAMPLES

Preparation of cosmetic body pigment

### Example 1

Mica (mean particle diameter 5µm, 200g) is suspended in 2000 ml of water, and the suspension is heated to 75 °C while stirring. A 30 % aqueous solution of hydrochloric acid is added to the suspension to adjust its pH value at 2. Further, TiO₂ fine particles (mean particle diameter 250 nm, 86 g, surface treated with aluminum, 'TIPAQUE CR-50' manufactured by ISHIHARA SANGYO Ltd.) are added to the suspension while stirring. After stirring for about 30 minutes, the pH value of the suspension is raised to pH 5 by means of sodium hydrate. The suspension is filtered, washed with deionized water, and dried (110 °C) to obtain a cosmetic body pigment. The amount of added TiO₂ fine particles is 30 wt% with respect to the total amount of the cosmetic body pigment, and it is considered that almost all of the added TiO₂ fine particles are deposited because the filtrate solution was transparent.

### Example 2

Mica (mean particle diameter 5µm, 50g) is suspended in 1000 ml of water, and the suspension is heated to 75 °C while stirring. A 30 % aqueous solution of hydrochloric acid is added to the suspension to adjust its pH value at 2. Further, TiO₂ fine particles (mean particle diameter 250 nm, 12.5 g, surface treated with aluminum, 'TIPAQUE CR-50' manufactured by ISHIHARA SANGYO Ltd.) are added to the suspension while stirring. After stirring for about 30 minutes, the pH value of the suspension is raised to pH 5 by means of sodium hydrate. The suspension is filtered, washed with deionized water, and dried (110 °C) to obtain a cosmetic body pigment. The amount of added TiO₂ fine particles is 20 wt% with respect to the total amount of the cosmetic body pigment, and it is considered that almost all of the added TiO₂ fine particles are deposited because the filtrate solution was transparent.

### Example 3

Mica (mean particle diameter 5µm, 50g) is suspended in 1000 ml of water, and the suspension is heated to 75 °C while stirring. A 30 % aqueous solution of hydrochloric acid is added to the suspension to adjust its pH at 2. Further, TiO₂ fine particles (mean particle diameter 250 nm, 50 g, surface treated with aluminum, 'TIPAQUE CR-50' manufactured by ISHIHARA SANGYO Ltd.) are added to the suspension under stirring. After stirring for about 30 minutes, the pH value of the suspension is raised to pH 5 by means of sodium hydrate. The suspension is filtered, washed with deionized water, and dried (110 °C) to obtain a cosmetic body pigment. The amount of added TiO₂ fine particles is 50 wt% with respect to the total amount of the cosmetic body pigment, and it is considered that almost all of the added TiO₂ particles were deposited because the filtrate solution was transparent.

### Evaluation of skin feel

A friction tester (KES-SE-DE, manufactured by KATOTECH Co., Ltd.) was used to evaluate an supredability (MIU), which is an alternative of the skin feel, of examples 1 to 3 and a commercially available conventional pigment.

**Table 1**

| Evaluation of extensibility | |
|---|---|
| | MIU |
| Example 1 (30 %) | 0.513 |
| Example 2 (20 %) | 0.561 |
| Example 3 (50 %) | 0.490 |
| Mica | 0.876 |
| TiO₂ | 0.782 |
| MP-1005 | 0.786 |
| Low luster pigment | 0.525 |
| Body-W | 0.548 |

The MIU represents a friction coefficient measured by the friction tester (KES-SE-DE, manufactured by KATOTECH). Lower MIU values indicate higher supredability (higher shipping property).

The mica is the one used as the raw material in examples 1 to 3. The TiO₂ is the one used as the raw material in examples 1 to 3. MP-1005 is a TiO₂ coated mica (manufactured by MERCK) in which mica is directly coated with TiO₂ through a hydrolysis of aqueous solution of a titanium salt with a conventional wet-preparing process. The Low luster pigment is a TiO₂ and BaSO4 coated mica (manufactured by MERCK) (see JP-A-60-94464) in which mica is directly coated through a hydrolysis and salt forming reaction using aqueous solutions of a titanium salt and a barium salt with a wet-preparing process, and Body-W is mica (manufactured by MERCK) (see JP-A-1-272668) in which coarse TiO₂ particles are obtained through a hydrolysis of a titanium salt in advance and then mica is added thereinto to make TiO₂ fine particles deposited thereon, and thereafter the mica is further coated with TiO₂ fine particles.

### Preparation of measurement specimen

1 g of various pigments are mixed and stirred in 9 g of inkimedium to obtain a mixed solution. The mixed solution is applied on a sheet of the black-and-white hiding paper by using bar-coater No. 20. The sheet was dried by wind to obtain a measurement specimen (D. D. C. (Draw Down Card)). Thus obtained measurement specimen is evaluated for blue color and a soft focus effect.

### Evaluation of blue color

A b* value was measured as an index to indicate the hue of blue. Larger b* value indicates less blue color.
(Table 2)

**Table 2**

| Measurement result of b* value | | | | | |
|---|---|---|---|---|---|
| | 15° | 25° | 45° | 75° | 110° |
| Example 1 (30 %) | -4.59 | -5.70 | -6.61 | -7.55 | -7.22 |
| Example 2 (20 %) | -4.88 | -6.23 | -7.40 | -8.59 | -8.25 |
| Example 3 (50 %) | -4.75 | -5.64 | -6.27 | -7.02 | -6.84 |
| MP-1005 | -7.70 | -6.92 | -8.42 | -11.83 | -15.41 |
| Low luster pigment | -13.32 | -12.87 | -11.60 | -9.57 | -9.13 |
| Body-W | -7.56 | -8.53 | -10.98 | -12.29 | -12.28 |

The b* values of D.D.C. (Draw Down Card) were measured with a variable angle differential colorimeter (X-RiTE MA 68) against the black color as the back ground color.
The result proves that the cosmetic body pigment of the present invention has larger values than other commercial pigments indicating less blue color.

### Evaluation of soft focus effect

Table 3 shows diffuse reflectance (Y) at incident light angle 45°for varying viewing angles.
[table 3]

**Table 3**

| Diffuse reflectance (Y) for varying viewing angles | | | | | |
|---|---|---|---|---|---|
| measurement angle | 0° | 15° | 30° | 45° | 60° |
| Example 1 (30 %) | 13.5 | 14.7 | 25.7 | 53.7 | 24.5 |
| Example 2 (20 %) | 10.4 | 12.0 | 23.9 | 53.6 | 24.1 |
| Example 3 (50 %) | 18.6 | 20.0 | 29.2 | 50.7 | 26.7 |
| Mica | 1.0 | 2.7 | 19.5 | 68.4 | 21.6 |
| MP-1005 | 16.7 | 32.8 | 63.3 | 95.6 | 47.9 |
| (incidence angle : 45°) | | | | | |

Table 3 shows the diffuse reflectance values (Y) of D.D.C. (Draw Down Card) measured with a digital varying angle gloss meter (UGV-5D SUGA TEST INSTRUMENTS) against the black color as the background color.
The result proved that the cosmetic body pigment of the present invention show less variation in Y values for varying viewing angles than other pigments and thus a soft focus effect is confirmed.

### Measurement of center-to-center distances of TiO₂ fine particles to be scattered

Six nearest neighboring particles with respect to a focused particle are selected and their distances are measured for averaging on a SEM photography, and then another focused particle is arbitrary selected to measure mean distances of its six nearest neighbors as before and similar measurements are repeated on 20 focused particles to calculate means based on the measurements. The mean interparticle distances for examples 1 to 3 are shown in Table 4.
[Table 4]

**Table 4**

| Interparticle distance | |
|---|---|
| | Interparticle distance(from center of particle) |
| Example 1 (30 %) | 700 nm |
| Example 2 (20 %) | 960 nm |
| Example 3 (50 %) | 400 nm |

Processing Example 1 (for cosmetics use)

| (Use example of compact powder) | |
|---|---|
| Talc | 50 weight parts |
| Pigments of examples 1 to 3 | 25 weight parts |
| Color pigments | 5 weight parts |
| Isopropyl myristate a suitable amount Magnesium stearate | 2 weight parts |

| [Foundation formulation 1] | |
|---|---|
| Talc | 38 weight parts |
| Pigments of examples 1 to 3 | 25 weight parts |
| Mica (8µ) | 10 weight parts |
| Magnesium stearate | 3 weight parts |
| Nylon powder 12 | 8 weight parts |
| Yellow iron oxide | 1.9 weight parts |
| Red iron oxide | 0.8 weight parts |
| Titanium oxide | 1.0 weight parts |
| Mineral oil | a suitable amount |
| (caprylic acid, capric acid) triglyceride | 3.3 weight parts |
| Butylparaben | 0.1 weight parts |

| [Foundation formulation 2] | |
|---|---|
| Talc | 49.0 weight parts |
| Magnesium stearate | 3.0 weight parts |
| Nylon powder | 15.0 weight parts |
| Red iron oxide | 0.9 weight parts |
| Yellow iron oxide | 2.4 weight parts |
| Black iron oxide | 0.4 weight parts |
| Pigments of examples 1 to 3 | 20.0 weight parts |
| Squalane | 4.0 weight parts |
| Dimethyl polysiloxane | 0.8 weight parts |
| Methylphenyl polysiloxane | 3.0 weight parts |
| Liquid paraffin | 1.2 weight parts |
| Paraben | a suitable amount |
| Anti-oxidant | a suitable amount |
| Perfume | a suitable amount |

### Processing Example 2 (use for resin composition ) [Use example for plastics]

| | |
|---|---|
| High density polyethylene (pellets) | 100 weight parts |
| Pigments of examples 1 to 3 | 1 weight parts |
| Magnesium stearate | 0.1 weight parts |
| Zinc stearate | 0.1 weight parts |

These are dry-blended to be injection molded.

The cosmetic body pigment achieved by the present invention has a good skin feel, little blue color, a high soft focus effect (angle dependency of reflected light intensity is small) and also has a color tone natural to skin and, as a result, it also has an appropriate hiding power (wrinkle hiding effect) and thus has preferable effects as the body pigment for cosmetics. Moreover, the invention can be used as fillers for resins.

## Claims

1. A cosmetic body pigment, **characterized in that** TiO₂ fine particles are deposited separately one another with form of mono layer on the surface of a thin-platelet like substrate.

2. The cosmetic body pigment according to claim 1, **characterized in that** a mean particle diameter of said TiO₂ fine particles is 150 to 450 nm.

3. The cosmetic body pigment according to claim 1 or 2, **characterized in that** particle diameters of said TiO₂ fine particles are not smaller than 100 nm.

4. The cosmetic body pigment according to any one of claims 1 to 3, **characterized in that** said TiO₂ fine particles are either untreated or surface treated with one or more hydroxides and/or hydrated oxide of which the principal component is an oxide of one or more metals selected from the group consisting of Si, Al, Zn, and Fe.

5. The cosmetic body pigment according to any one of claims 1 to 4, **characterized in that** the amount of said TiO₂ fine particles is 15 to 55 wt% of the total amount of the cosmetic body pigment.

6. The cosmetic body pigment according to any one of claims 1 to 5, **characterized in that** a mean center-to-center distance between TiO₂ particles among six neighboring particles is not smaller than one and a half times the mean particle diameter of said TiO₂ fine particles.

7. The cosmetic body pigment according to any one of claims 1 to 6, **characterized in that** a mean particle diameter of said thin-platelet like substrate is 0.5 to 50 µm and a mean thickness of the same is 0.01 to 1 µm.

8. The cosmetic body pigment according to any one of claims 1 to 7, **characterized in that** said thin-platelet like substrate is mica.

9. The cosmetic body pigment according to any one of claims 1 to 8, **characterized in that** said TiO₂ fine particles are deposited onto the surface of said thin-platelet like substrate by surface electrical charge attractive forces.

10. A process for preparing a cosmetic body pigment comprising following steps;
suspending thin-platelet like substrates in water and adding TiO₂ fine particles thereto while stirring;
depositing said TiO₂ fine particles onto the surface of a thin-platelet like substrate by changing the pH value of the suspension containing said thin-platelet like substrates and said TiO₂ fine particles; and
collecting solids components of said suspension by filtering and washing followed by drying them.

11. A cosmetic composition comprising cosmetic body pigments according to any one of claims 1 to 9.

12. A resin composition comprising cosmetic body pigments according to any one of claims 1 to 9.
